## Europäisches Patentamt
## European Patent Office
## Office européen des brevets

(19)

(11) Numéro de publication: **0 066 497**
**B1**

(12)

# FASCICULE DE BREVET EUROPEEN

(45) Date de publication du fascicule du brevet:
**24.07.85**

(51) Int. Cl.⁴: **A 61 B 6/00**

(21) Numéro de dépôt: **82400904.7**

(22) Date de dépôt: **14.05.82**

(54) **Support de source de rayonnement à orientation multiple.**

(30) Priorité: **27.05.81 FR 8110561**

(43) Date de publication de la demande:
**08.12.82 Bulletin 82/49**

(45) Mention de la délivrance du brevet:
**24.07.85 Bulletin 85/30**

(84) Etats contractants désignés:
**DE IT NL**

(56) Documents cités:
**EP - A - 0 049 562**
**DE - A - 2 120 344**
**FR - A - 2 247 952**
**FR - A - 2 342 704**
**US - A - 2 822 477**

(73) Titulaire: **THOMSON-CSF, 173, Boulevard Haussmann,
F-75379 Paris Cedex 08 (FR)**

(72) Inventeur: **Le Sonn, Marcel, THOMSON-CSF
SCPI 173, bld. Haussmann, F-75379 Paris Cedex 08 (FR)**

(74) Mandataire: **Barbin le Bourhis, Joel et al,
THOMSON-CSF SCPI 173, boulevard Haussmann,
F-75379 Paris Cedex 08 (FR)**

## Description

L'invention concerne un support de source de rayonnement à orientation multiple, dont la source de rayonnement est en fonctionnement, associée à un détecteur de ce rayonnement. Les applications de la présente invention concernent plus particulièrement le domaine de la radiologie.

On trouve, notamment en radiodiagnostic, différents types de support de source de rayonnement X, associés à une table d'examens, comportant au moins un détecteur de rayonnement X, sur laquelle repose un patient à examiner. Au cours d'un même examen, il est souvent nécessaire qu'un axe de travail, constitué par l'axe d'un faisceau de rayonnement émis par une source, telle qu'un ensemble radiogène situé d'un côté du patient, et le centre d'un détecteur, tel qu'un film radiographique, situé de l'autre côté du patient, soit obtenu sous des incidences variées; ceci exige que le support de l'ensemble radiogène, permette à ce dernier d'occuper toutes les positions dans lesquelles son orientation réalise l'incidence désirée.

L'état actuel de la technique comporte des supports de source de rayonnement qui ne permettent qu'imparfaitement ces conditions de travail, et qui de plus présentent un certain nombre d'inconvénients. On peut citer notamment:

a) un support constitué d'une colonne, supportant au-dessus d'une table d'examen, un bras horizontal et transversal à cette table; ce bras comporte un chariot, sur lequel est monté un ensemble radiogène, qui est ainsi capable d'un déplacement le long de ce bras.

Les inconvénients majeurs de ce type de support sont d'une part, le manque de rigidité du bras, étant donné la longueur qui lui est imposée pour permettre le travail en incidence latérale avant, et d'autre part, la gêne permanente procurée par l'extrémité de ce bras, vis-à-vis des utilisateurs.

b) Un autre type de support utilise également un bras comme ci-dessus cité, mais qui selon sa longueur nécessaire au-dessus de la table d'examen, peut coulisser dans le haut de la colonne qui le supporte: l'extrémité de ce bras gêne ainsi beaucoup moins les utilisateurs, ce bras n'étant en position avancée, que pour les incidences latérales avant.

L'inconvénient reste dans son manque de rigidité, lors de ces incidences latérales avant; d'autre part, un autre inconvénient réside en ce que ce bras, coulissant dans la colonne, exige derrière celle-ci un espace plus important, qui pénalise les locaux de dimensions réduites.

Il faut souligner qu'avec ces deux types de support, il est impossible d'obtenir un faisceau de rayonnement dont l'axe soit horizontal.

c) Il existe aussi un type de support, ayant une forme semicirculaire, et comportant à une extrémité un ensemble radiogène; un tel support est installé de manière à permettre à l'ensemble radiogène, de décrire une trajectoire circulaire, transversale à la table d'examen et au-dessus de celle-ci, en fonction du mouvement de ce support. Ce type de support permet d'obtenir des incidences latérales avant, sans présenter les inconvénients précédemment cités pour les modèles précédents; par contre, pour obtenir un axe de rayonnement horizontal, ce support doit tourner d'environ 125°, et son extrémité opposée à celle de l'ensemble radiogène, pénètre profondément dans l'espace de la table d'examen. Ceci constitue une gêne considérable, et parfois même un empêchement à l'utilisation d'éléments radiologiques complémentaires, disposés sous cette table d'examen.

La demande de brevet allemand DE-A-2 120 344 décrit un dispositif pour examen radiologique, dans lequel, une table de radiologie est associée à un support portant à une extrémité une source de rayonnement X et à l'autre extrémité un moyen détecteur de ce rayonnement. Le support a une forme générale semi-circulaire et comporte une partie droite, à laquelle est fixée la source de rayonnement par l'intermédiaire de moyens permettant des mouvements de rotation de cette source de rayonnement. Le dispositif comporte en outre des moyens permettant des mouvements du support dans un plan transversal à celui de la table de radiologie et dans un plan parallèle à cette dernière. Cependant, il est à remarquer que dans ce dispositif le support porte le moyen détecteur et pénêtre profondément dans l'espace de la table de radiologie.

La demande de brevet français publiée sous N° 2 342 704 décrit un support mobile pour appareil de radiodiagnostic. Le support mobile a également une forme semi-circulaire, et porte à une extrémité un tube à rayons X et, à l'autre extrémité un moyen détecteur. Ce support mobile est associé à une couchette d'examen et peut effectuer un mouvement de rotation sur lui-même, dans un plan transversal à celui de la couchette d'examen. Cette rotation s'effectue selon 90° de part et d'autre d'une position centrale, et l'on peut remarquer que dans cette position centrale, le support pénêtre profondément dans l'espace situé sous la couchette d'examen.

La présente invention concerne un support de source de rayonnement, dont l'agencement lui permet de présenter une gêne minimum, dans toutes les positions qu'il peut occuper. Cet agencement permet en outre à une source de rayonnement, d'émettre un faisceau de rayonnement sous toutes les incidences nécessaires en radiologie, et notamment:

– des incidences longitudinales,
– des incidences latérales,
– des incidences composées (par la combinaison des deux incidences précédentes),
– des rayonnements horizontaux.

Selon l'invention, un support de source de rayonnement à orientation multiple, associé en fonctionnement, à une table de radiologie située transversalement à un axe horizontal y–y, comportant:

– une source de rayonnement, munie de moyens de mouvement, associée en fonctionnement, à au moins un détecteur de ce rayonnement,

– des premiers moyens permettant un premier mouvement de ce support dans un plan transversal à la table de radiologie,

– des seconds moyens moteurs permettant un second mouvement dudit support autour de l'axe horizontal Y–Y,

– ledit support étant constitué d'une partie droite, portant la source de rayonnement, solidaire d'une partie courbe, est caractérisé en ce que la partie courbe coopère avec des moyens moteurs contenus dans un coffret, pour assurer un déplacement de cette partie courbe entrainant le premier mouvement du support, afin de permettre à la source de rayonnement, d'émettre des faisceaux de rayonnement, sous des incidences latérales, différentes, et en ce que la partie droite comporte des moyens de déplacement grâce auxquels elle coopère avec un chariot, pour permettre le déplacement de la source de rayonnement le long de cette partie droite afin d'obtenir un faisceau de rayonnement dont l'axe soit horizontal et ajustable en hauteur, et en ce que le détecteur de rayonnement est indépendant au support.

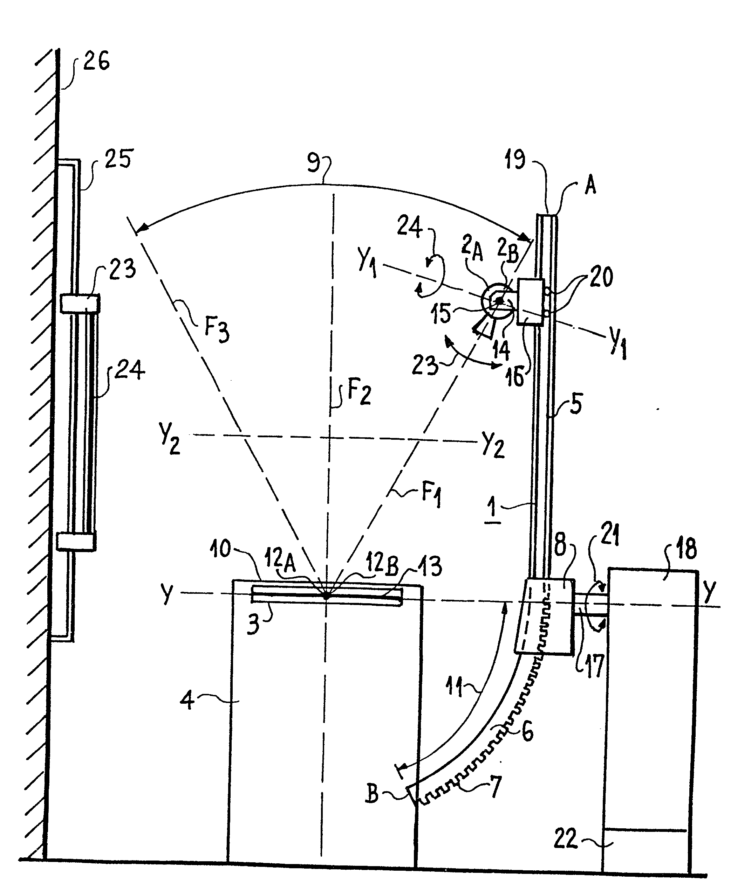

L'invention sera mieux comprise à l'aide de la description qui va suivre, et de la figure unique jointe.

La figure représente schématiquement par une vue de côté, un support selon l'invention, ainsi que des moyens extérieurs avec lesquels il coopère.

La figure montre un support 1 selon l'invention, supportant une source de rayonnement 2A, qui dans l'exemple non limitatif de la description, est un ensemble radiogène 2B; celui-ci est associé en fonctionnement, à au moins un détecteur de rayonnement, qui étant donné la nature de la source, et un porte-film radiographique 3, contenu dans une table 4 d'examen radiologique; celle-ci, dont le plan supérieur 10 est horizontal, est disposée transversalement à un axe y–y, lui-même horizontal. Le porte-film radiographique 3, contient un film radiographique 13, disposé selon un plan horizontal, affleurant l'axe y–y, et dont le centre est un point 12A, sur cet axe y–y.

Le support 1, est constitué par une partie droite 5, solidaire d'une partie courbe 6, représentant un arc de cercle, qui dans l'exemple non limitatif de cette description, a un angle d'ouverture de 65° (cette ouverture pouvant être ajustée en fonction des caractéristiques recherchées); ce support 1 est soutenu par un coffret 8, au niveau de la partie courbe 6, dans une position telle, que le centre 12B de cet arc de cercle coïncide avec le point 12A.

La partie courbe 6, comporte un moyen quelconque, tel que par exemple dans la description, une crémaillère 7, grâce à laquelle elle coopère avec des moyens moteurs, contenus dans le coffret 8, pour aussurer son déplacement et par là, celui du support 1.

Ce premier mouvement du support 1, s'effectue dans un plan transversal à la table 4 autour du point 12A; son amplitude est déterminée par l'angle d'ouverture de la partie courbe 6, qui selon l'exemple précédemment décrit, permet d'une part, à la partie droite 5, d'occuper une position verticale, et à son extrémité A, de décrire une trajectoire le long de la flèche 9, dans les limites de celle-ci, et d'autre part à l'extrémité B, de décrire une trajectoire le long de la flèche 11 et dans les limites de celle-ci.

Le coffret 8 est solidaire d'un moyen, tel que par exemple un arbre 17, disposé selon l'axe y–y, par lequel il est lié à un pilier 18; ce cernier comporte des moyens coopérant avec l'arbre 17, afin de permettre à celui-ci, et au coffret 8 d'effectuer un mouvement de rotation autour de l'axe y–y, selon la flèche 21.

Ce mouvement de rotation du coffret 8, provoque un second mouvement du support 1, qui s'exécute sur un plan parallèle à l'axe dans le coffret 8, afin d'assurer le mouvement circulaire de cette partie courbe autour du point 12B.

D'autre part, l'agencement du support 1, grâce à la partie droite 5 qu'il comporte, coopérant avec le chariot 16, sur lequel est monté l'ensemble radiogène 2B, permet à ce dernier d'être déplacé le long de cette partie droite; ceci permet à l'ensemble radiogène 2B, quand la partie droite 5 est en position verticale, d'être orienté de manière à émettre un faisceau de rayonnement dont l'axe est horizontal, à des hauteurs variables et aisément ajustables.

Dans ce cas, l'ensemble radiogène 2B, est généralement associé à un détecteur situé à l'extérieur de la table 4, et dont le plan de réception est vertical, comme par exemple un porte-film radiographique 23 contenent un film radiographique 24: ce porte-film radiographique 23 peut être déplacé verticalement le long de montants 25, fixés contre un mur 26 de la salle de radiologie, afin d'être positionné à une hauteur quelconque, en fonction du type d'examen à réaliser. L'ajustement de la position de l'ensemble radiogène 2B, le long de la partie droite 5, permet de centrer l'axe $y_2$–$y_2$ du faisceau de rayonnement sur le plan de réception du film radiographique 24, quelle que soit la position de celui-ci, en maintenant constante la distance entre ce dernier et l'ensemble radiogène 2B.

Un tel agencement permet également à un support selon l'invention, de présenter un encombrement réduit et une gêne minimum, tant pour les équipements que pour les utilisateurs; on peut remarquer à cet égard, que la position du support 1, nécessaire à une incidence latérale arrière (axe de rayonnement F1), est la même que celle qui permet d'obtenir un faisceau de rayonnement d'axe horizontal, et que dans cette position, L'extrémité B pénètre peu dans l'espace de la table 4.

Ainsi que précédemment expliqué, ce second mouvement du support 1, provoqué par la rotation du coffret 8 autour de l'axe y–y, est exécuté dans un plan transversal au plan de son premier mouvement, et parallèle à l'axe longitudinal de la table 4; par suite longitudinal (non représenté) de la table 4, et transversal au plan du premier mouvement du support 1, autour de l'axe y–y.

L'ensemble radiogène 2B, maintenu par un étrier 14, est capable d'un premier mouvement de rotation, autour d'un axe représenté par le pivot 15, selon la flèche 23. L'étrier 14 est rendu solidaire d'un chariot 16, par un axe non visible sur le dessin, place selon un axe $Y_1$–$Y_1$; l'ensemble radiogène 2B est capable d'un second mouvement de rotation autour de cet axe, selon la flèche 24.

La partie droite 5 et le chariot 16 comportent des moyens assurant le déplacement manuel ou motorisé de ce dernier, le long de la partie droite 5; ces moyens étant par exemple des gorges 19 dans lesquelles circulent des roulements 20.

Les mouvement de rotation dont est capable l'ensemble radiogène 2B, lui permettent d'être orienté, quelle que soit la position qu'il occupe le long de la partie droite 5, de manière à ce que l'axe F1, d'un faisceau de rayonnement qu'il émet, passe par le centre 12a du film radiographique 13.

Le premier mouvement dont est capable le support 1, permet à l'ensemble radiogène 2B, d'occuper une infinité de positions, selon une trajectoire parallèle à la flèche 9; par rapport à sa position centrale, pour laquelle l'axe du faisceau de rayonnement qu'il émet correspond à l'axe F2, ses positions extrêmes lui permettent d'émettre un faisceau de rayonnement selon des axes F1 et F3; ces dernier représentant chacun un angle de 30° par rapport à l'axe F2, ce qui est généralement suffisant pour les incidences latérales.

Il faut remarquer, que pour toutes les positions que peut occuper l'ensemble radiogène 2B, le long de la trajectoire dont il est capable, l'axe du faisceau de rayonnement qu'il émet intersecte toujours un même point, le point 12B, qui dans l'exemple décrit coïncide avec le centre 12A du film radiographique 13.

Cette caractéristique très avantageuse est obtenue, grâce à l'agencement du support 1, dont la partie courbe 6 comporte un moyen qui est la crémaillère 7, coopérant avec les moyens contenus de ce mouvement qui permet d'effectuer des incidences longitudinales, pour toutes les positions occupées par l'ensemble radiogène 2B, l'axe d'un faisceau de rayonnement émis par celui-ci, intersecte également toujours le point 12A.

Ceci permet en conservant toujours le même point d'intersection avec le plan du film radiographique 13, de réaliser des incidences complexes, en combinant le premier et le second mouvement dont est capable le support 1.

Cette nouvelle caractéristique d'un support selon l'invention est obtenue grâce à un moyen que comporte le coffret 8, tel que l'arbre 17 coopérant avec le pilier 18 pour obtenir la rotation de ce coffret et, par là, celle du support 1, autour de l'axe y–y.

Le pilier 18 est monté sur un chariot 22, coopérant avec un chemin de roulement (non représenté) afin de permettre le déplacement, du pilier 18 et de l'ensemble qu'il supporte, le long de la table 4, parallèlement à l'axe longitudinal de cette dernière.

Cette possibilité de déplacement du pilier 18, permet de reconstituer l'ensemble des mouvements précédemment décrits, en différentes positions le long de la table 4.

Un support de source de rayonnement à orientation multiple, selon l'invention, peut être intégré dans des équipements, ou être utilisé s'il est autonome, associé à des tables d'examens, basculantes ou non.

**Revendications**

1. Support de source de rayonnement (2A) à orientation multiple, associé en fonctionnement à une table (4) de radiologie, située transversalement à un axe horizontal y–y, comportant:
   – une source de rayonnement (2A), munie de moyens de mouvement (15), associée en fonctionnement, à au moins un détecteur (3) de ce rayonnement,
   – des premiers moyens permettant un premier mouvement de ce support (1) dans un plan transversal à la table (4) de radiologie,
   – des seconds moyens moteurs permettant un second mouvement dudits support (1) autour de l'axe horizontal Y–Y,
   – ledit support (1) étant constitué d'une partie droite (5), portant la source de rayonnement (2A), solidaire d'une partie courbe (6), caractérisé en ce que la partie courbe (6) coopère avec des moyens moteurs contenus dans un coffret (8), pour assurer un déplacement de cette partie courbe (6), entrainant le premier mouvement du support (1), afin de permettre à la source de rayonnement (2A), d'émettre des faisceaux de rayonnement, sous des incidences latérales, différentes, et en ce que la partie droite (5) comporte des moyens de déplacement (19) grâce auxquels elle coopère avec un chariot (16), pour permettre le déplacement de la source de rayonnement (2A) le long de cette partie droite (5), afin d'obtenir un faisceau de rayonnement dont l'axe soit horizontal et ajustable en hauteur, et en ce que le détecteur (3) de rayonnement est indépendant du support (1).

2. Support selon la revendication 1, caractérisé en ce que la partie courbe (6) est constituée par un arc de cercle, dont le centre (12B) coïncide avec le centre (12A), d'un film radiographique (13), sur l'axe y–y, permettant à l'axe du faisceau émis par la source de rayonnement de toujours intersecter le centre (12B) dans les mouvements du support (1).

3. Support selon l'une des revendications précédentes, caractérisé en ce que la partie courbe (6), comporte une crémaillère (7), grâce à laquelle elle coopère avec les moyens moteurs contenus dans le coffret (8) pour assurer le premier mouvement du support (1).

4. Support selon l'une des revendications précédentes, caractérisé en ce que le coffret (8) comporte un arbre (17), grâce auquel il coopère avec les seconds moyens moteurs contenus dans un pilier (18), pour assurer le second mouvement du support (1) et permettre des incidences longitudinales.

5. Support selon l'une des revendications précédentes, caractérisé en ce que la combinaison du premier et du second mouvement dont est capable le support (1), permet d'obtenir des incidences complexes.

6. Support selon la revendication 4, caractérisé en ce que le pilier (18) est monté sur un chariot (22), afin de permettre le déplacement du pilier (18) et de l'ensemble qu'il supporte, le long de la table (4), parallèlement à l'axe longitudinal de cette dernière.

## Patentansprüche

1. Träger für eine Strahlungsquelle (2A) mit Mehrfachorientierung, die im Betrieb einem Radiologietisch (4) zugeordnet ist, der transversal zu einer Horizontalachse y–y angeordnet ist, enthaltend:

– eine Strahlungsquelle (2A), die mit Bewegungsmitteln (15) versehen ist und im Betrieb wenigstens einem Detektor (3) für diese Strahlung zugeordnet ist,

– erste Mittel, die eine erste Bewegung dieses Trägers (1) in einer zu dem Radiologietisch (4) transversalen Ebene gestatten,

– zweite Antriebsmittel, welche eine zweite Bewegung dieses Trägers (1) um die waagerechte Achse Y–Y gestatten,

– wobei der Träger (1) aus einem geraden Teil (5) gebildet ist, welcher die Strahlungsquelle (2A) trägt, und fest verbunden ist mit einem gekrümmten Teil (6), dadurch gekennzeichnet, dass der gekrümmte Teil (6) mit Antriebsmitteln zusammenwirkt, die in einem Gehäuse (8) enthalten sind, um eine Verschiebung dieses gekrümmten Teiles (6) zu gewährleisten, welche die erste Bewegung des Trägers (1) nach sich zieht, um zu gestatten, dass die Strahlungsquelle (2A) Strahlungsbündel unter verschiedenen seitlichen Einfallswinkeln aussendet, und dass der gerade Teil (5) Verschiebungsmittel (19) umfasst, durch welche er mit einem Wagen (16) zusammenwirkt, um die Verschiebung der Strahlungsquelle (2A) längs dieses geraden Teiles (5) zu gestatten, damit ein Strahlungsbündel erhalten wird, dessen Achse horizontal und in der Höhe einstellbar ist, und dass der Strahlungsdetektor (3) unabhängig von dem Träger (1) ist.

2. Träger nach Anspruch 1, dadurch gekennzeichnet, dass der gekrümmte Teil (6) durch einen Kreisbogen gebildet ist, dessen Zentrum (12B) mit dem Zentrum (12A) eines radiographischen Films (13) auf der Achse y–y zusammenfällt, wodurch es ermöglicht wird, dass die Achse des von der Strahlungsquelle abgegebenen Bündels stets das Zentrum (12B) bei den Bewegungen des Trägers (1) schneidet.

3. Träger nach einem der vorstehenden Ansprüche, dadurch gekennzeichnet, dass der gekrümmte Teil (6) eine Zahnstange (6) umfasst, durch welche er mit den Antriebsmitteln zusammenwirkt, die in dem Gehäuse (8) enthalten sind, um die erste Bewegung des Trägers (1) zu gewährleisten.

4. Träger nach einem der vorstehenden Ansprüche, dadurch gekennzeichnet, dass das Gehäuse (8) eine Welle (17) umfasst, durch welche es mit den zweiten Antriebsmitteln zusammenwirkt, die in einem Pfosten (18) enthalten sind, um die zweite Bewegung des Trägers (1) zu gewährleisten und longitudinale Einfallswinkel zu ermöglichen.

5. Träger nach einem der vorstehenden Ansprüche, dadurch gekennzeichnet, dass die Kombination der ersten mit der zweiten Bewegung, welche der Träger (1) ausführen kann, die Erzielung von komplexen Einfallswinkeln ermöglicht.

6. Träger nach Anspruch 4, dadurch gekennzeichnet, dass der Pfosten (18) auf einem Wagen (22) montiert ist, um die Bewegung dieses Pfostens (18) und der von ihm getragenen Einheit längs des Tisches (4) parallel zur Längsachse desselben zu ermöglichen.

## Claims

1. Support for a radiation source (2A) of multiple orientation associated in operation with a radiology table (4) situated transversely to a horizontal axis y–y, comprising:

– a radiation source (2A) equipped with movement means (15) and associated in operation with at least one detector (3) for said radiation,

– first means permitting a first movement of said support (1) in a plane transverse to the radiology table (4),

– second drive means permitting a second movement of said support (1) about the horizontal axis Y–Y,

– said support (1) being constituted by a straight portion (5) carrying the radiation source (2A) and integral with a curved portion (6), characterized in that the curved portion (6) cooperates with drive means contained in a housing (8) to ensure a displacement of said curved portion (6) resulting in the first movement of the support (1) in order to permit the radiation source (2A) to emit radiation beams at different lateral angles of incidence, and that the straight portion (5) comprises displacement means (19) by which it cooperates with a carriage (16) to permit the displacement of the radiation source (2A) along said straight portion (5) in order to obtain a radiation beam whouse axis is adjustable horizontally and in height, and that the radiation detector (3) is independent of the support (1).

2. Support according to claim 1, characterized in that the curved portion (6) is formed by a circular arc whose centre (12B) coincides with the centre (12A) of a radiographic film (13) on the axis y–y, thus permitting the axis of the beam emitted by the radiation source always to intersect the centre (12B) in the movements of the support (1).

3.Support according to any one of the preceding claims, characterized in that the curved portion (6) comprises a rack (6) via which it cooperates with the drive means contained in the housing (8) to ensure the first movement of the support (1).

4. Support according to any one of the preceding claims, characterized in that the housing (8) comprises a shaft (17) via which it cooperates with the second drive means contained in a column (18) to ensure the second movement of the support (1) and permit longitudinal angles of incidence.

5. Support according to any one of the preceding claims, characterized in that the combination of the first and the second movement of which the support (1) is capable permits complex angles of incidence to be obtained.

6. Support according to claim 4, characterized in that the column (18) is mounted on a carriage (22) in order to permite the movement of said column (18) and the assembly which it supports along the table (4) parallel to the longitudinal axis thereof.

1/1